# EUROPEAN PATENT SPECIFICATION

(11) **EP 1 942 182 B1**
(45) Date of publication and mention of the grant of the patent: **04.04.2012**
(21) Application number: 06820022.9
(22) Date of filing: 03.10.2006
(51) Int. Cl.: C12N 5/00, C12N 5/09, C12N 7/00

(54) **VERO CELL LINE WHICH IS ADAPTED TO GROW IN SUSPENSION, METHOD OF OBTAINING SAME AND VIRAL VACCINES**
AN DAS WACHSTUM IN SUSPENSION ANGEPASSTE VERO-ZELLINIE, VERFAHREN ZUR GEWINNUNG DAVON UND VIRALE IMPFSTOFFE
LIGNEE CELLULAIRE VERO CONVENANT A UNE CROISSANCE EN SUSPENSION, PROCEDE D'OBTENTION AFFERENT ET VACCINS VIRAUX

(30) Priority: 04.10.2005 US 723377 P
(43) Date of publication of application: 09.07.2008
(73) Proprietor: Zelltek S.A., 3000 Santa Fe - Santa Fe (AR)
(72) Inventor: DAELLI, Marcelo Gustavo, 3000 Santa Fe - Santa Fe (AR); FORNO, Angela Guillermina, Santa Fe (AR); KRATJE, Ricardo, Santa Fe (AR); ETCHEVERRIGARAY, Marina, Santa Fe (AR); PAILLET, Cristian, Santa Fe (AR)
(74) Representative: Gislon, Gabriele
(86) International application number: PCT/ES2006/070143
(87) International publication number: WO 2007/039662

(56) References cited:
- EP-A- 1 260 581
- WO-A1-2005/063970
- YUNG-SHYENG TSAO ET AL: "Development and improvement of a serum-free suspension process for the production of recombinant adenoviral vectors using HEK293 cells" CYTOTECHNOLOGY, KLUWER ACADEMIC PUBLISHERS, DO, vol. 37, no. 3, 1 November 2001 (2001-11-01), pages 189-198, XP019236726 ISSN: 1573-0778
- LITWIN J.: 'The growth of vero cells in suspension as cell aggregates in serum.free media' CYTOTECHNOLOGY vol. 10, no. 2, 1992, pages 169 - 174, XP009124301
- FRAZZATI-GALLINEA ET AL.: 'Vero-cell rabies vaccine produced using serum-free medium' VACCINE vol. 23, no. 4, 09 December 2004, pages 511 - 517, XP003013076
- JINDRICH CINATL J.R.: 'Protein free culture of Vero cells: a substrate for replication of human pathogenic viruses' CELL BIOL. INT. vol. 17, no. 9, September 1993, pages 885 - 896, XP003013077
- CHOY Y.: 'Inactivated hantaan virus vaccine derived from suspension culture of vero cells' VACCINE vol. 21, no. 17-18, 16 May 2003, pages 1867 - 1873, XP003013078

## Description

### TECHNICAL FIELD OF THE INVENTION

The invention concerns to a cell line that can be grown in a culture free of fetal calf serum (FCS) and in suspension without a carrier, and to production of viral vaccines using said cell line. More particularly, the present invention concerns to the establishment of a cell line that can be grown in a culture free of proteins and in suspension without need of the cells to be adhered to any carrier, and a process for producing viral vaccines with said cell line. The present invention further relates to the viruses obtained using the inventive method and to the vaccines formulated with said viruses.

### BACKGROUND OF THE INVENTION

For the production of viral vaccines chicken eggs, mouse brains, primary cells or established cell lines are used in order to propagate the virus (Principles of Virology. Molecular Biology, pathogenesis and control, 2001). These conventional techniques have several problems:
1) The use of chicken eggs requires management of chicken breeding, management of fertilized eggs to be adjusted to a vaccine production schedule, and laborious procedures including extensive purification for completely removing components derived from egg proteins (Tree et al., 2001).
2) Cell lines require in general the addition of fetal calf serum as a cell growth factor. Therefore, there is an associated risk of contamination with infection agents and prions, and moreover, high-quality FCS is very expensive, a fact that adds a considerable cost to the vaccine.
3) Among established cell lines where various kinds of viruses can be propagated, Vero cells (African Green Monkey Kidney Epithelial Cells) are widely used for production of high quality vaccines for humans and animals (Butler et al., 2000; Franzzati-Gallina et al., 2001; US Patent N° 4,664,912; Montagnon, 1989; Montagnon et al., 1981). Furthermore, Vero cells are the only recommended cells to prepare viruses for vaccine production by reverse genetics in the document: "WHO guidance on development of influenza vaccine reference viruses by reverse genetics". 2005.6, page 3.
However, Vero cells have a tendency to adhere to surfaces. Thus, the use of Vero cells for large-scale cultures is, due to the need of reactors with very large surface or to the need of introduction of cell-carriers in the culture, extremely costly.

Fetal calf serum is often used for the propagation of mammalian cell lines. However, when mammalian cells are used for the production of recombinant proteins or virus production, there is increasing pressure to remove serum from the manufacturing process. Some of the driving reasons to implement serum-free cell-culture technology are the expense of serum, the variability between serum lots and serum quality, regulatory concerns regarding biological agents in serum and the burden of removing serum proteins in downstream processing. There is also a recognized need, for reasons of reduced cost and process scale up, for use of cell lines adapted to grow in suspension. There is an urgent need to develop more economic processes for the production of quality vaccine at a low-cost in order to make them available worldwide.

Many developments have been carried out to obtain Vero cell lines in suspension to obtain a virus for vaccine manufacturing (US 4,664,912; US 4,525,349: US 5,719,051; Higher production of rabies virus in serum-free medium cell cultures on microcarriers. Frazzati-Gallina NM. J Biotechnol. 2001 Dec 14;92(1):67-72. However, these documents describe, in general, the use of carriers and microcarriers to obtain suspensions of anchorage-depending Vero cell lines.

In the state of the art, it has never been reported a Vero cell line to obtain a virus for vaccine manufacturing, adapted to growing in suspension, in the absence of supporting materials for its adherence (carriers or microcarriers), adapted to grow in a culture medium free of fetal calf serum, and capable of growing also in a protein-free medium. On the other hand, there are references to Vero cell lines which may grow in suspensions forming cell aggregates ("The growth of Vero cells in suspension as cell-aggregates in serum-free media". Cytotechnology. 1992;10(2):169-74. Litwin J.). Litwin describes a method to grow Vero cells in suspension as cell aggregates in serum free media. However, cell aggregates are difficult to infect with viruses and therefore they are not adequate for vaccine production (as can be read in the patent application US 2005153419-A). There are no reports of Vero cells that may grow in suspension as individual cells without forming aggregates in high density cultures.

In order to obviate the disadvantages of the prior art as stated above, this invention has now established a novel Vero cell line which can be grown without FCS, without protein and in suspension comprising isolated cells in the absence of cell aggregates. This cell line can be used in large scale production of vaccines in standard fermentation reactors in the absence of supporting materials for its adherence (without the need of any kind of cell supporting, such as carriers or microcarriers in the culture medium). The individual cells suspensions have known advantages over the aggregates suspension, for those skilled in the art, for example improves of efficiency in virus production systems that involve high density bioreactors. The present invention also establishes a process for producing a viral vaccine culturing Vero cells in suspension without FCS and then infecting the cells with the virus to be used in the vaccine production. The vaccine so produced is of low cost and high safety profile.

### SUMMARY OF THE INVENTION

The Vero cell line, subject matter of the present invention, useful for the production of viruses for vaccine formulation, is adapted to grow in suspension, in the absence of supporting materials for its adherence and in a culture free of fetal calf serum. Furthermore, this cell line is adapted to grow in a protein-free culture. This cell line also is adapted to grow in a suspension as isolated cells, in the absence of cell aggregates. Specifically, this cell line is deposited in the German Collection of Microorganisms and Cell Cultures (DSMZ, Braunschweig, Germany) under the name of sVero p66 and whose number assigned by the International Deposit Authority is DSM ACC2791.

In another preferred embodiment of this invention, it is disclosed a procedure to obtain a virus for vaccine formulation, which is constituent of another purpose of this invention, comprising the following steps:
a. growing a Vero cell line adapted to grow in suspension, in the absence of supporting materials for its adherence and, preferably, in the absence of proteins, up to a concentration from 1x10⁵ to 30x10⁶ cells per ml of culture medium,
b. infecting the cell line of step "a" with said virus with an optimum multiplicity of infection (M.O.I.) from 0.001 to 10, and
c. collecting the virus produced during the infection, preferably by centrifugation.

In preferred embodiments of this invention, said virus is selected from the group consisting of the human immunodeficiency virus, such as HIV-1 and HIV-2; polio virus; hepatitis A virus, human coxsackie virus; rhinovirus; echovirus; equine encephalitis virus; rubella virus, dengue viruses, encephalitis virus, yellow fever virus, coronavirus, vesicular stomatitis virus, rabies virus, ebola virus, parainfluenza virus, mumps virus, measles virus, respiratory syncytial virus, influenza virus, Hantaan virus, bunga virus, hemorrhagic fever virus, reovirus, rotavirus, parvoviruses, papilloma virus, polyoma virus, adenovirus, herpes simplex virus (HSV) 1 and 2, varicella zoster virus, cytomegalovirus (CMV), smallpox virus, vaccinia virus, pox viruses, African swine fever virus and the unclassified agent of delta hepatitis, and the agents of non-A, non-B hepatitis. Preferably said virus is selected from the group consisting of polio virus, rabies virus, yellow fever virus, hepatitis A virus and influenza virus.

### DESCRIPTION OF THE DRAWING

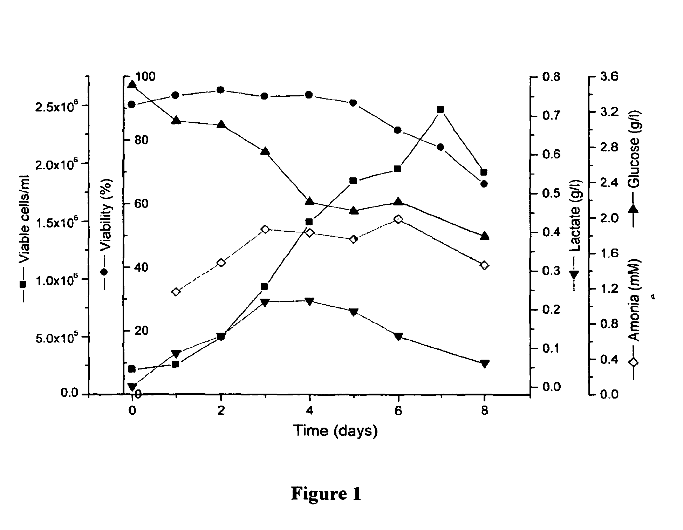

FIG. 1: shows growth of Vero E6 AGS according to the present invention.

### DETAILED DESCRIPTION OF THE INVENTION

The Vero cell line, subject matter of the present invention, useful for the production of viruses for vaccine formulation, is adapted to grow in suspension, in the absence of supporting materials for its adherence (carriers or microcarriers) and in a culture free of fetal calf serum. Furthermore, this cell line is adapted to grow in a protein-free culture. This cell line is also adapted to grow in a suspension as isolated cells, in the absence of cell aggregates. Specifically, said cell line is deposited in the German Collection of Microorganisms and Cell Cultures (DSMZ, Braunschweig, Germany) under the name of sVero p66 and whose number assigned by the International Deposit Authority is DSM ACC2791.

The procedure for obtaining said Vero cell line adapted to grow in suspension, in the absence of supporting materials for its adherence and in a culture free of fetal calf serum and free of proteins comprises the following steps:
a. adapting an anchorage-depending Vero cell line, preferably the Vero E6 cell line (passage 26, Banco Argentino de Células), to a culture with a low content of fetal calf serum by a gradual adjustment of the culture from a high concentration of fetal calf serum from 7 to 15% to a culture of low concentration of fetal calf serum from 0 to 3%, preferably 2%;
b. applying the Vero cell line obtained in the previous step "a." to a culture in suspension of the same culture medium of low concentration fetal calf serum from 0 to 3%, preferably 2%, for enough time, at least 30 days, so that said cell line be adapted to grow in suspension in the absence of supporting materials for its adherence. That is achieved with an abrupt passage of the cells from T-flasks to spinner flasks, without any carrier, and gradually replacing remaining serum supplementation by serum free media containing proteins and growth factors. During the initial phase of suspension culture adaptation the cells tend to form aggregates of 2-30 cells, especially at cell densities higher than 1.10⁶ cells/ml.; and
c. adapting said cell line obtained in step "b" to grow in a medium free of fetal calf serum and free of proteins. After several passages, and replacing step "b" medium by protein free media, no cell clumping is observed, and Vero E6 is considered to be adapted to the growth in a suspension culture in protein free media, and that cellular clone is denominated "Vero E6 AGS".

Vero E6 AGS cells have been shown to maintain an average doubling time of 24 hours in continuous growth culture over a period of at least one month and have retained the susceptibility to be infected by several viruses.

Additionally, there was not difference in the cellular response (as measured by viability and doubling time) to the growth in mediums with low concentration of proteins and growth factors and to the growth in mediums free of proteins (protein free media).

The Vero E6 AGS cell line has been deposited in the German Collection of Microorganisms and Cell Cultures (DSMZ, Braunschweig, Germany) under the name sVero p66 and whose number, assigned by the International Deposit Authority, is DSM ACC2791.

### Suspension Culture and Infection

Cells are maintained in suspension in 30 to 1,000 ml cultures in 50 to 1,500 ml glass spinner flasks (Techne, UK) agitated at 50-80 rpm in a 5% CO₂ incubator at 33-37 °C.

During cell maintenance, cultures are kept in exponential phase by diluting cells approximately every two days in order to maintain cell densities from 1.10.⁵ cells/ml to 12.10.⁵ cells/ml. For infection experiments, exponentially growing cells are centrifuged and resuspended in fresh culture media at a cell density of 1.10⁶ cells/ml.

Infection is initiated by adding concentrated viruses to the cultures at an optimum multiplicity of infection (M.O.I.) of 0.001 to 10. A 500-1,500 mu.l cell suspension aliquot is transferred in sterile tubes at different times post infection. The different aliquots are stored at -80 °C for further analyses. Viral particles are recovered from the total of the frozen samples by 3 freeze-thaw cycles.

According to the present invention, cells grow individually in suspension and are properly infected by different virus producing an appropriate yield of the antigenic viral particles suitable for vaccine production.

The viruses that may be produced by application of the present invention are selected from the group consisting of the human immunodeficiency virus, such as HIV-1 and HIV-2; polio virus; hepatitis A virus, human coxsackie virus; rhinovirus; echovirus; equine encephalitis virus; rubella virus, dengue viruses, encephalitis virus, yellow fever virus, coronavirus, vesicular stomatitis virus, rabies virus, ebola virus, parainfluenza virus, mumps virus, measles virus, respiratory syncytial virus, influenza virus, Hantaan virus, bunga virus, hemorrhagic fever virus, reovirus, rotavirus, parvoviruses, papilloma virus, polyoma virus, adenovirus, herpes simplex virus (HSV) 1 and 2, varicella zoster virus, cytomegalovirus (CMV), smallpox virus, vaccinia virus, pox viruses, African swine fever virus and the unclassified agent of delta hepatitis, and the agents of non-A, non-B hepatitis. Preferably the polio virus, the rabies virus, the yellow fever virus, hepatitis A virus and the influenza virus.

The viruses that can be obtained according to the present invention are useful for vaccine formulation using methods known by those skilled in the art of vaccine formulation. Said viruses or the viral antigens are concentrated and purified using methods known in the art such as centrifugation and chromatography. The vaccines that can be obtained by the present invention comprise said viruses or viral antigens, at least one diluent and, optionally, adjuvants, excipients and chemotherapeutic agents known in the art. If necessary, said viruses will undergo an attenuation or inactivation process.

The present invention will now be described in more details through the following application examples showing preferred forms to practice this invention. However, said examples are provided for illustration purposes only, to better understand the present invention, and do not intend to limit the scope of the present invention.

### EXAMPLE 1

More specifically, the Vero cell line of the present invention was prepared using the following procedures:

Vero cells from the line Vero E6 (passage 26, Banco Argentino de Células) were used as the starting material. Cells were cultured in a monolayer culture flask of 25 cm², containing MEM (GIBCO BRL, USA) medium supplemented with 10% FCS. Incubation was carried out at 37°C in a 5% CO₂ atmosphere. After sufficient expansion of the cells, the cells were then cultured in the above medium containing 20% serum-free medium and subcultured upon confirming no abnormality in cells. After subculture and sufficient expansion of the cells, the culture medium was replaced with a medium containing 40% serum-free medium and sub-cultured upon confirming no abnormality in cells. The procedure was repeated until the content of serum-free medium was of 80% (2% FCS). At this point cells were trypsinated and inoculated (2.5.10⁵ cells/ml) in a spinner flask (Techne, UK) containing the same medium. Agitation was at 50 rpm and incubation was at 37°C in a 5% CO₂ atmosphere. The cells were transfer to fresh medium according to the evolution of the pH of the culture. After 90 days of the initiation of the adaptation procedure, cells were seeded in a medium containing half a volume of MEM supplemented with 0.2% FCS and half a volume of ExCell^{™} 302 CHO serum-free (JRH Biosciences, USA) medium supplemented with 2 mM glutamine. Duplication period in this medium was about 24 hrs with a viability of 98-100%, after a week of the transfer. Cells were grouped in clusters of less than ten cells when the cell concentration was superior to 1.5.10⁶ cells/ml. At this point, the half volume of MEM was replaced with the protein free SMIF-6 medium (GIBCO BRL, USA). After this, the proportion of ExCell^{™} 302 CHO serum-free medium was diminished and 120 days after of the initiation of the adaptation procedure, Vero cells (Vero E6) were growing in suspension in 100% protein free SMIF-6 medium. These cells were named "Vero E6 AGS" for Vero E6 adapted to grow in suspension as isolated cells without cells aggregates, without carriers.

For storage, Vero E6 AGS cells in exponential growth period were centrifuged at 1000 rpm and the cell pellet suspended in a solution containing 45% of SMIF-6 conditioned medium, 45% of fresh SMIF-6 medium and 10% of dimethylsulfoxide (DMSO). This cell suspension was fractionated in cryotubes (cryogenic tubes) containing 1ml of the suspension each one and frozen in steps of: 1h at 4°C, 1h at -20°C in nitrogen atmosphere and finally in liquid nitrogen at -196°C.

### EXAMPLE 2

### Susceptibility of Vero E6 AGS cells to viral infection

### Susceptibility of Vero E6 AGS cells to viral infection

The susceptibility of Vero E6 AGS cells to viral infection was assayed using the vesicular stomatitis virus (VSV, Indiana strain: ATCC VR-1238). Titration of the virus was performed using Vero E6 cells (passage 31). For this, 4.10⁴ cells were seeded in each well of a 96 wells titration plate (GREINER, GERMANY). After 3 hrs of incubation at 37°C in a 5% CO2 atmosphere, cells were infected with different amounts of a viral suspension. After 24 hs viral titer was estimated as the maximal viral dilution with capacity to produce cytopathic in 100% of the cells.

For virus production, 3.2.10⁵ Vero E6 AGS cells were seeded in a spinner flask containing SMIF-6 medium and were infected with 0.01 cytopathic units/ml of the viral suspension. Table 1 shows that after 48 hours all the cells have been killed by the virus. Viral titer in the supernatant was 1 cytopathic unit/ml. This result indicates that in average Vero E6 AGS cells are able to amplify 100 times the seeded virus in the conditions here given as an example.

**TABLE 1**

| Time after infection (hs) | % of viable cells | Viral titer (cytopathic units/ml) |
|---|---|---|
| 0 | 97 | 0.0001 |
| 24 | 37 | ND |
| 48 | 0 | 0.01 |

### EXAMPLE 3

The relationship between cell density and viral production was studied using the Vero E6 AGS cell line and the VSV virus. For this, Vero E6 AGS were grown in suspension until density 0.75.10⁶ or 5. 10⁶ cells/ml and infected with VSV virus at a multiplicity of infection (M.O.I.) of 0.1.
Culture medium: SMIF 6
Additives: FCS 2%
Culture condition: 37°C, 5% CO2
Spinner rotation: 50 rpm
Cellular density at the beginning of culture: about 1.10⁵ cells/ml
Virus: VSV
MOI: 0.1

Samples were taken 20 hrs after infection and viral titer was measured in accordance with a plaque counting method.

The sample dilution that produces cytopathic effect in 50% of the inoculated cultures, contains 1 Tissue Culture Infection Dose 50% (TCID 50). The determination of this value was in accordance with the Reed-Muench procedure (Reed and Muench, 1938).
Table.2 shows that the viral titers rose in an approximately proportional form to the cell density.

**TABLE.2**

| Cell Density | Viral Titer | Viral Titer/ Cell Density |
|---|---|---|
| (cells/ml) | (TCID50/ml) | (TCID50/cell) |
| 0.75.10⁶ | 4.64.10⁸ | 618 |
| 2.44.10⁶ | 3.16.10⁹ | 1295 |

### EXAMPLE 4

Vero E6 AGS was used to propagate Herpes Simplex (HSV):
Culture conditions were as follows:
   Cellular density at infection: 2.0.10⁵
   MOI: 0.002
   Culture scale: 100 ml spinner flask
   Culture medium: SMIF 6 and SMIF 6 / FCS 2%
   Spinner rotation: 50 rpm
   Culture conditions: 37°C, 5%CO2
   Duration of culture: 2 days

Table.3 shows that the HSV virus, a DNA virus, was produced using Vero E6 AGS in suspension in the absence of microcarriers and in a protein-free culture medium. It was observed a suspension of isolated individual cells without cell aggregates. It is also observed that the viral titration is slightly higher in the experiment conducted in the absence of FCS.

**TABLE.3**

| Culture medium | HSV titer (TCID50/ml) |
|---|---|
| SMIF 6 | 5.88.10⁸ |
| SMIF 6 + FCS (2%) | 4.64.10⁸ |

### EXAMPLE 5

Vero E6 AGS was used to propagate Poliovirus (OPV type I Sabin strain). Culture conditions were as follows:
Cellular density at infection: 3.0.10⁵
MOI: 1
Culture scale: 100 ml spinner flask
Culture medium: SMIF 6 and SMIF 6 / FCS 2%
Spinner rotation: 50 rpm
Culture conditions: 34°C, 5%CO2
Duration of culture: 1 day

**TABLE.4**

| Culture medium | OPV titer (TCID50/ml) |
|---|---|
| SMIF 6 | 2.5.10⁸ |
| SMIF 6 + FCS (2%) | 1.8.10⁸ |

### References

### Patent documents

| | | | |
|---|---|---|---|
| 1. | US 4,664,912 | Wiktor et al. | May, 1987 |
| 2. | US 6,825,036; | Makizumi, et al. | September 3, 2002 |
| 3. | US 6,656,719; | Gould, et al.; | October 21, 1998 |
| 4. | US 6,656,720; | Groner. et al.; | Jul. 12, 2002 |
| 5. | US 6,146,873; | Kistner, et al. | October 15, 1997 |
| 6. | US 6,008,036; | Fanget, et al.; | May 22, 1998 |
| 7. | US 5,824,536; | Webster, et al.; | June 17, 1996 |
| 8. | US 5,719,051; | Mundt, et al.; | December 13, 1994 |
| 9. | US 4,783,407; | Provost, et al.; | September 30, 1985 |
| 10. | US 4,664,912; | Wiktor, et al.; | October 1, 1984 |
| 11. | BR 0002694; | Gallina Neuza Frazatti; | June 19, 2000 |
| 12. | US 4,525,349; | Montagnon, et al.; | December 29, 1981 |
| 13. | US 20050019928; | Rasty, Siyamak; et al. | August 17, 2004 |
| 14. | US 20040137013; | Katinger, Hermann; et al.; | October 3, 2003 |
| 15. | US 20010001709; | Lau, Allan S.; | December 13, 2000 |
| 16. | US 2005013419; | Liu, Zhong; et al. | July 14, 2005 |

### Papers

1. Vero-cell rabies vaccine produced using serum-free medium. Frazatti-Gallina NM, et al. Vaccine. 2004 Dec 9;23(4):511-7.R
2. Rabies virus production in high Vero cell density cultures on macroporous microcarriers. Yokomizo AY.Biotechnol Bioeng. 2004 Mar 5;85(5):506-15.
3. Optimization of virus yield as a strategy to improve rabies vaccine production by Vero cells in a bioreactor. Trabelsi K J Biotechnol. 2006 Jan 24;121(2):261-71.
4. Inactivated Hantaan virus vaccine derived from suspension culture of Vero cells. Choi Y. Vaccine. 2003 May 16;21(17-18):1867-73.
5. High immunogenic enterovirus 71 strain and its production using serum-free microcarrier Vero cell culture. Liu CC, Vaccine. 2006 Aug 2.
6. Immunogenicity and protective efficacy in monkeys of purified inactivated Vero-cell SARS vaccine. Qin E, Vaccine. 2006 Feb 13;24(7):1028-34.
7. The growth of Vero cells in suspension as cell-aggregates in serum-free media. Litwin J. Cytotechnology. 1992;10(2):169-74.
8. Studies on the efficiency of measles virus antigen production using Vero cell culture in a microcarrier system. Mendonca RZ. Braz J Med Biol Res. 1994 Jul;27(7):175-87.
9. Preparation and evaluation of Vero-cell infectious bursal disease vaccine in Pakistan. Rasool MH,Hussain I. Vaccine. 2006 Apr 5;24(15):2810-4.
10. Development of a purified, inactivated, dengue-2 virus vaccine prototype in Vero cells: immunogenicity and protection in mice and rhesus monkeys. Putnak R. J Infect Dis. 1996 Dec;174(6):1176-84.
11. Thousand litre scale microcarrier culture of Vero cells for killed polio virus vaccine. Promising results. Montagnon B. Dev Biol Stand. 1983;55:37-42.
12. A novel process for production of hepatitis A virus in Vero cells grown on microcarriers in bioreactor. Sun MB. World J Gastroenterol. 2004 Sep 1;10(17):2571-3.
13. Development of a novel influenza vaccine derived from a continuous cell line. Kistner O. ALTEX. 2001;18(1):50-4.
14. Production of influenza virus in cell cultures for vaccine preparation. Merten OW. Adv Exp Med Biol. 1996;397:141-51.
15. Development of a Vero cell-derived influenza whole virus vaccine. Kistner O. Dev Biol Stand. 1999;98:101-10.
16. Production of influenza virus in serum-free mammalian cell cultures. Merten OW. Dev Biol Stand. 1999;98:23-37.
17. Higher production of rabies virus in serum-free medium cell cultures on microcarriers. Frazzati-Gallina NM. J Biotechnol. 2001 Dec 14;92(1):67-72.
18. A purified inactivated Japanese encephalitis virus vaccine made in Vero cells. SrivastavaAK. Vaccine. 2001 Aug 14;19(31):4557-65.
19. Development of a mammalian cell (Vero) derived candidate influenza virus vaccine. Kistner O. Vaccine. 1998 May-Jun;16(9-10):960-8.
20. Industrial-scale production of inactivated poliovirus vaccine prepared by culture of Vero cells on microcarrier. Montagnon BJ. Rev Infect Dis. 1984 May-Jun;6 Suppl 2:S341-4.
21. Application of a serum-free medium for the growth of Vero cells and the production of reovirus. Butler M. Biotechnol Prog. 2000 Sep-Oct;16(5):854-8.
22. Inactivated Hantaan virus vaccine derived from suspension culture of Vero cells. Y Choi. Vaccine, May 16,2003; 21(17-18): 1867-73.
23. Cell aggregate suspension culture for large-scale production of biomolecules. Tolbert WR, Hitt MM, Feder J. In Vitro. 1980 Jun;16(6):486-90.
24. Alterations in the growth and adhesion pattern of Vero cells induced by nutritional stress conditions. Genari SC. Cell Biol Int. 1998;22(4):285-94.
25. Vero-cell rabies vaccine produced using serum-free medium. Neuza M. Frazatti-Gallina. Vaccine Volume 23, Issue 4 , 9 December 2004, Pages 511-517.
26. Higher production of rabies virus in serum-free medium cell cultures on microcarriers. Neuza M. Frazzati-Gallina. Journal of Biotechnology Volume 92, Issue 1,15 November 2001, Pages 67-72.
27. Protein-free culture of Vero cells: a substrate for replication of human pathogenic viruses. Jindrich Cinatl, Jr. Cell Biology International Volume 17, Issue 9 , September 1993, Pages 885-896.
28. Butler, M; Burgener, A.; Patrick, M; Berry, D.; Moffatt, D.; Huzel, N.; Bernabé, N. And Coombs, K. (2000). Applicaton of a serum-free medium for the growth of Vero Cells and production of reovirus. Biotechnol. Progr., 16, 854-858.
29. Frazzati-Gallina, N.M.; Paoli, R.L.; Mourao-Fuches, R.M.; Jorge, S.A.C and Pereira, C.A. (2001). Higher production of rabies virus in serum-free medium cell cultures on microcarriers. J Biotechnol., 92, 67-72.
30. Montagnon, B.J. ; Fanget, B. ; Nicolas, A.J. (1981). Large scale cultivation of Vero cells in microcarrier culture for virus vaccine production. Preliminary results for killed poliovirus vaccine. Dev. Biol. Stand., 47, 55-64.
31. Montagnon, B.J. (1989) Polio and rabies vaccines produced in continuous cell lines: a reality for Vero cell line. Dev. Biol. Stand., 70, 27-47.
32. Tree, J.A.; Richardson, C., Fooks, A.R.; Clegg, J.C.; Looby, D. (2001) Comparison of large-scale mammalian cell culture systems with egg culture for the production of influenza virus A vaccine strains. Vaccine, 19, 3441-3450
33. Reed, L.J., Muench, H. (1938), A simple method of estimating fifty percent end points. J. Hyg., 27, 493-497.

## Claims

1. A VERO cell line adapted to grow in suspension, in the absence of supporting, materials for its adherence and in a culture medium free of fetal calf serum wherein said line is the line deposited in the German Collection of Microorganisms and Cell Cultures (DSMZ, Braunschweig, Germany) under the name sVero p66 and whose number assigned by the International Deposit Authority is DSM ACC2791.

2. The cell line of claim 1 wherein the cell line is adapted to grow in a protein-free culture medium.

3. The cell line of claim 1 wherein said suspension comprises isolated cells in the absence of cell aggregates.

4. The cell line of claim 1 wherein said line is useful for virus production for vaccine formulation.

5. A procedure to produce virus to formulate vaccines wherein the procedure comprises the steps of:
a. growing the VERO cell line adapted to grow in suspension of claim 1, up to a cell concentration from 1x10⁵ to 30x10⁶ cells per ml of culture medium;
b. infecting the cell line of step "a" with said virus with an optimum multiplicity of infection from 0.001 to 10; and
c. collecting the virus production produced during the infection.

6. The procedure of claim 5 wherein said VERO cell line adapted to grow in suspension of claim 1, is adapted to grow in a culture medium free of fetal calf serum and free of proteins.

7. The procedure of claim 5 wherein said virus is selected from the group consisting of the human immunodeficiency virus, such as HIV-1 and HIV-2; polio virus; hepatitis A virus, human coxsackie virus; rhinovirus; echovirus; equine encephalitis virus; rubella virus, dengue viruses, encephalitis virus, yellow fever virus, coronavirus, vesicular stomatitis virus, rabies virus, ebola virus, parainfluenza virus, mumps virus, measles virus, respiratory syncytial virus, influenza virus, Hantaan virus, bunga virus, hemorrhagic fever virus, reovirus, rotavirus, parvoviruses, papilloma virus, polyoma virus, adenovirus, herpes simplex virus (HSV) 1 and 2, varicella zoster virus, cytomegalovirus (CMV), smallpox virus, vaccinia virus, pox viruses, African swine fever virus and the unclassified agent of delta hepatitis, and the agents of non-A, non-B hepatitis.

8. The procedure of claim 5 wherein said virus is the polio virus.

9. The procedure of claim 5 wherein said virus is the rabies virus.

10. The procedure of claim 5 wherein said virus is the hepatitis A virus.

11. The procedure of claim 5 wherein said virus is the yellow fever virus.

12. The procedure of claim 5 wherein said virus is the influenza virus.

13. The procedure of claim 5 wherein said step "c" comprises a centrifugation.

## Patentansprüche

1. VERO-Zelllinie, welche dazu angepasst ist, in Suspension, in Abwesenheit von Trägermaterialien für dessen Haftung und in einem Kulturmedium frei von fetalem Kälberserum zu wachsen, wobei die genannte Linie die in der Deutschen Sammlung von Mikroorganismen und Zellkulturen (DSMZ, Braunschweig, Deutschland) unter dem Namen sVero p66 hinterlegte Linie ist und dessen von der Internationalen Hinterlegungsbehörde zugewiesenen Nummer DSM ACC2791 ist.

2. Zelllinie nach Anspruch 1, wobei die Zelllinie dazu angepasst ist, in einem proteinfreien Kulturmedium zu wachsen.

3. Zelllinie nach Anspruch 1, wobei die genannte Suspension isolierte Zellen in Abwesenheit von Zellaggregaten umfasst.

4. Zelllinie nach Anspruch 1, wobei die genannte Linie für die Virusproduktion für de Impfstoffformulierung ist geeignet.

5. Verfahren für die Virusproduktion um Impfstoffe zu formulieren, wobei das Verfahren folgende Schritte umfasst:
a. die VERO-Zelllinie nach Anspruch 1, welche dazu angepasst ist, in Suspension zu wachsen, bis zu einer Zellkonzentration von 1x10⁵ bis 30x10⁶ Zellen pro ml Kulturmedium wachsen zu lassen;
b. die Zelllinie von Schritt "a" mit dem genannten Virus mit einer optimalen Multiplizität der Infektion von 0,001 bis 10 zu infizieren; und
c. die während der Infektion produzierte Virusproduktion zu sammeln.

6. Verfahren nach Anspruch 5, wobei die genannte VERO-Zelllinie nach Anspruch 1, welche dazu angepasst ist, in Suspension zu wachsen, dazu angepasst ist, in einem Kulturmedium frei von fetalem Kälberserum und frei von Proteinen zu wachsen.

7. Verfahren nach Anspruch 5, wobei das genannte Virus aus der Gruppe gewählt wird, die aus Folgendem besteht: dem humanen Immunodefizienz-Virus, wie HIV-1 und HIV-2; Polio-Virus; Hepatitis-A-Virus, humanen Coxsackie-Virus; Rhinovirus; ECHO-Virus; equine Enzephalitis-Virus; Röteln-Virus, Dengue-Viren, dem Enzephalitis-Virus, Gelbfiebervirus, Coronavirus, vesikulären Stomatitis-Virus, Tollwut-Virus, Ebola-Virus, Parainfluenzavirus, Mumpsvirus, Masernvirus, Respiratorischen Synzytial-Virus, Influenzavirus, Hantaan-Virus, Bunga-Virus, hämorrhagischen Fieber-Virus, REO-Virus, Rotavirus, Parvoviren, dem Papilloma-Virus, Polyoma-Virus, Adenovirus, Herpes Simplex-Virus (HSV) 1 und 2, Varizella Zoster-Virus, Cytomegalie-Virus (CMV), Variola-Virus, Vaccinia-Virus, Pockenviren, dem afrikanischen Schweinepest-Virus und dem unklassifizierten Erreger der Delta-Hepatitis, und die Erreger der Non-A-Non-B Hepatitis.

8. Verfahren nach Anspruch 5, wobei das genannte Virus das Polio-Virus ist.

9. Verfahren nach Anspruch 5, wobei das genannte Virus das Tollwut-Virus ist.

10. Verfahren nach Anspruch 5, wobei das genannte Virus das Hepatitis-A-Virus ist.

11. Verfahren nach Anspruch 5, wobei das genannte Virus das Gelbfiebervirus ist.

12. Verfahren nach Anspruch 5, wobei das genannte Virus das Influenzavirus ist.

13. Verfahren nach Anspruch 5, wobei der genannte Schritt "c" eine Zentrifugation umfasst.

## Revendications

1. Une lignée cellulaire VERO adaptée pour pousser dans une suspension, à défaut de matériaux de support pour y adhérer et dans un milieu de culture libre de sérum de veau foetal où cette lignée est celle déposée dans la Collection allemande de microorganismes et cultures de cellules (DSMZ, Braunschweig, Allemagne) sous le nom sVero p66 et dont le numéro lui ayant été assigné par l'Autorité Internationale de dépôt est DSM ACC2791.

2. La lignée cellulaire de la revendication 1 où la lignée cellulaire est adaptée pour pousser dans un milieu de culture libre de protéines.

3. La lignée cellulaire de la revendication 1 où cette suspension comporte des cellules isolées à défaut d'agrégats de cellules.

4. La lignée cellulaire de la revendication 1 où cette lignée est utile pour la production de virus pour formuler des vaccins.

5. Une méthode pour produire des virus pour formuler des vaccins, cette méthode comportant les étapes suivantes:
a. faire pousser la lignée cellulaire VERO adaptée pour pousser dans la suspension de la revendication 1 jusqu'à une concentration cellulaire de 1x10⁵ a 30x10⁶ de cellules par ml de milieu de culture;
b. infecter la lignée cellulaire de l'étape "a" avec ce virus ayant une multiplicité optimale d'infection de 0,001 à 10; et
c. cueillir la production de virus obtenue durant l'infection.

6. La méthode de la revendication 5 où cette lignée cellulaire VERO adaptée pour pousser dans la suspension de la revendication 1 est adaptée pour pousser dans un milieu de culture libre de sérum de veau foetal et libre de protéines.

7. La méthode de la revendication 5 où ce virus est sélectionné du groupe consistant en le virus d'immunodéficience humaine, tel que le VIH-1 et VIH-2; le virus de la polio; le virus de l'hépatite A, le virus de coxsakie humaine; le rhinovirus; l'échovirus; le virus d'encéphalite équine; le virus de la rubéole; les virus de la dengue; le virus de l'encéphalite, le virus de la fièvre jaune, le coronavirus, le virus de stomatite vésiculeuse, le virus de la rage, le virus de l'Ébola; le virus parainfluenza, le virus des oreillons, le virus de la rougeole, le virus respiratoire syncytial, le virus de la grippe; le virus du Hantaan, le virus du bunga; le virus de la fièvre hémorragique, réovirus, rotavirus, parvovirus, virus du papillome, le virus du polyome, l'adénovirus, le virus de l'herpès simplex (VHS) 1 et 2, le virus de la varicelle-zona, le cytomégalovirus (CMV), le virus de la variole, la virus de la vaccine; les virus de maladie pustuleuse, le virus de la peste porcine africaine et l'agent non classifié de l'hépatite delta et les agents des hépatites non A et non B.

8. La méthode de la revendication 5 où ce virus est le virus de la polio.

9. La méthode de la revendication 5 où ce virus est le virus de la rage.

10. La méthode de la revendication 5 où ce virus es le virus de l'hépatite A.

11. La méthode de la revendication 5 où ce virus est le virus de la fièvre jaune.

12. La méthode de la revendication 5 où ce virus est le virus de la grippe.

13. La méthode de la revendication 5 où cette étape "c" comporte une centrifugation.
